## Europäisches Patentamt

## European Patent Office

## Office européen des brevets

(19)

(11) Publication number: **0 152 103**

**B1**

(12) **EUROPEAN PATENT SPECIFICATION**

(45) Date of publication of patent specification: **14.12.88**

(51) Int. Cl.⁴: **C 07 C 69/54,** C 07 C 67/327

(21) Application number: **85101542.0**

(22) Date of filing: **13.02.85**

(54) Process for producing methacrylate esters by dehydration of 2-hydroxy-2-methylpropionate esters.

(30) Priority: **13.02.84 US 579569**

(43) Date of publication of application:
**21.08.85 Bulletin 85/34**

(45) Publication of the grant of the patent:
**14.12.88 Bulletin 88/50**

(84) Designated Contracting States:
**DE FR GB**

(56) References cited:
**GB-A-1 023 176**
**GB-A-1 041 702**

(73) Proprietor: **E.I. DU PONT DE NEMOURS AND COMPANY**
**1007 Market Street**
**Wilmington Delaware 19898 (US)**

(72) Inventor: **de Colibus, Raymond Lew**
**2498 Kenwood Lane**
**Bartlett Tennessee 38134 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

EP 0 152 103 B1

Courier Press, Leamington Spa, England.

## Description

### Field of the invention

This invention relates to an improvement in a process for making methacrylate esters.

### Background of the invention

Sulfuric acid has long been known as a dehydrating agent for hydroxy esters, converting them to the corresponding unsaturated esters. However, as pointed out by Volker, et al., in U.S. Patent 3,487,101, at column 1, lines 33 et seq., dehydration proceeds smoothly only with β hydroxy compounds where conventional dehydrating agents, such as $H_2SO_4$, can be used. In contrast, the dehydration of α-hydroxy esters meets with considerable difficulties, for decomposition generally occurs and, thus, yield of desired methacrylate ester is ordinarily very low. Sennewald, et al., in U.S. Patent 3,022,336, in column 1, lines 20 et seq., teaches that dehydrating α-hydroxy esters such as 2 - hydroxy - 2 - methylpropionates is very difficult since the molecule is unstable and prone to uncontrollable decomposition.

### Summary of the invention

Surprisingly, in this invention conditions have been found which, contrary to past teachings, give a high yield dehydration of α hydroxy ester.

A process has now been discovered in which 2 - hydroxy - 2 - methylpropionate esters can be dehydrated in nearly quantitative yields to produce methacrylate esters. The new process combines careful control of temperature, mole ratios of reactants, contact times and sulfuric acid dehydrating agent strength, to obtain quantitative yields of methacrylate esters.

Specifically, the process of this invention is a process for preparing a methacrylate ester which comprises contacting a lower alkyl ($C_1$—$C_8$) 2 - hydroxy - 2 - methylpropionate ester with sulfuric acid of 90% strength or more in water at a temperature of 0 to 50°C for 5 minutes or less, wherein the mole ratio of sulfuric acid to 2 - hydroxy - 2 - methylpropionate ester is between 5-50/1, followed by recovering the methacrylate ester formed.

### Detailed description of the invention

Dehydration yield losses increase as temperature is raised at a given reaction time and ratio of sulfuric acid to 2 - hydroxy - 2 - methylpropionate ester. Thus contact time of ester and sulfuric acid should be short and the reaction quenched as temperature approaches the upper range. Conversely, if desired, the temperature can be lowered and the contact times lengthened.

Mole ratio of $H_2SO_4$ to 2 - hydroxy - 2 - methylpropionate ester should be between 5-50:1, preferably 5-12/1. Again an interrelationship exists and the higher the ratio the shorter the time required for dehydration, all other factors being equivalent.

Dehydration of 2 - hydroxy - 2 - methylpropionate esters can occur at acid strengths of 90% $H_2SO_4$ or higher. As acid strength decreases below 100% $H_2SO_4$, progressively more stringent conditions are necessary for dehydration. These conditions of longer time and/or higher temperature lower yield. At acid strengths significantly above 100% reaction time is significantly reduced at a given temperature and such strengths are preferred.

### Examples

#### Example 1

16.96 g $H_2SO_4$ (101.8%, 176.2 m moles $H_2SO_4$) was added to a 25 ml flask equipped with a fluoropolymer coated stirring bar. 2.02 g methyl 2 - hydroxy - 2 - methylpropionate (MHiB) (also known as methyl α-hydroxyisobutyrate) 17.1 m moles, was then added at room temperature (22°C). The temperature rose to 51.5°C after one minute of mixing and a sample was taken after 1.4 minutes. Conversion after 1.4 minutes on a mole basis was

|  |  |
|---|---|
| 58.4% methyl methacrylate (MMA) | Total Yield |
| 24.5% methacrylic acid (MAA) | 82.9% |

Conversion after 5 minutes of stirring was

|  |  |
|---|---|
| 52.2% MMA | Total Yield |
| 44.5% MAA | 96.7% |

Conversion after 105 minutes at room temperature showed more MAA present but the total yield remained at 95+%.

Note the nearly quantitative yield.

    Mole ratio $H_2SO_4$/MHiB was 10.3

    Strength of $H_2SO_4$ was 101.8%

Time of contact was 5 minutes to maximum yield

EP 0 152 103 B1

Comparison A
Lower yield, with lower ratio H$_2$SO$_4$/MHiB

3.40 g H$_2$SO$_4$ (101.8%, 35.3 m moles H$_2$SO$_4$) was added to a 15 ml 2 neck flask equipped with a fluoropolymer coated stirring bar. 2.02 g MHiB (17.1 m moles) was then added at room temperature (22°C). The temperature rose to 48°C after 1.3 minutes. Conversion after 0.5 minutes on a mole basis was

| 21.8% MMA | } Total Yield |
|-----------|--------------|
| 0.4% MAA | 22.2% |

Conversion after 5 minutes was

| 32.4% MMA | } Total Yield |
|-----------|--------------|
| 1.2% MAA | 33.5% |

Conversion after 105 minutes was unchanged

| 32.8% MMA | } Total Yield |
|-----------|--------------|
| 1.7% MAA | 34.5% |

Temperature was 48°C
Mole ratio H$_2$SO$_4$/MHiB was 2.06.
Strength of H$_2$SO$_4$ was 101.8%

Time was about 5 minutes to maximum yield. Note that yield was much lower than in Example 1, primarily because the mole ratio H$_2$SO$_4$/MHiB was much lower.

Comparison B
Lower yield using lower strength H$_2$SO$_4$ and lower temperatures

52.65 g H$_2$SO$_4$ (94.2%, 506.1 m moles) was added to 5.96 g MHiB (49.5 m moles) at ambient (about 22°C) temperature in a vessel described as in Example 1.

Conversion at 5 minutes was

| 11.5% MMA | } Total Yield |
|-----------|--------------|
| 1.2% MAA | 12.7% |

Conversion after 60 minutes was

| 30.9% MMA | } Total Yield |
|-----------|--------------|
| 3.2% MAA | 34.1% |

Temperature was ambient. The Mole ratio H$_2$SO$_4$/MHiB was 10.2. The Strength of H$_2$SO$_4$ was 94.2%.

Example 2
Dehydration using butyl 2-hydroxy 2-methylpropionate

4.87 g H$_2$SO$_4$ (100.21%, 49.8 m moles) was added to a 15 ml 2 neck flask equipped with a teflon coated stirring bar. Then 0.81 g (5.06 m moles) of butyl 2 - hydroxy - 2 - methylpropionate (BHiB) was added at room temperature (26°C). The temperature rose to 41.5°C in one minute.

Conversion after 5 minutes (temperature was 35°C) on a mole basis was

| 66.1% | butyl methacrylate (BMA), |
|-------|---------------------------|
| 30.8% | BHiB unreacted |

**Claim**

A process for preparing methacrylate esters in at least 95% yield which comprises contacting a lower alkyl 2 - hydroxy - 2 - methylpropionate ester with sulfuric acid of 90% strength or higher in water at a temperature of between 0 and 50°C for 5 minutes or less, wherein the mole ratio of sulfuric acid to the lower alkyl 2 - hydroxy - 2 - methylpropionate is between 5-50/1.

3

**Patentanspruch**

Verfahren zur Herstellung von Methacrylatestern in einer Ausbeute von wenigstens 95%, umfassend das In-Berührung-Bringen eines Neideralkyl - 2 - hydroxy - 2 - methylpropionsäureesters mit Schwefelsäure einer Stärke von 90% oder höher in Wasser bei einer Temperatur zwischen 0 und 50°C für 5 Minuten oder weniger, wobei das Molverhältnis von Schwefelsäure zu dem Niederalkyl - 2 - hydroxy - 2 - methylpropionat zwischen 5-50/1 liegt.

**Revendication**

Un procédé pour préparer des esters méthacryliques en un rendement d'au moins 95%, qui consiste à mettre en contact un 2 - hydroxy - 2 - méthylpropionate d'alkyle inférieur avec de l'acide sulfurique titrant 90% ou plus dans l'eau, à une température de 0 à 50°C, pendant 5 minutes ou moins, le rapport molaire de l'acide sulfurique au 2 - hydroxy - 2 - méthylpropionate d'alkyle inférieur étant compris entre 5/1 et 50/1.